(19)

Europäisches
Patentamt
European
Patent Office
Office européen
des brevets

(11)     **EP 1 297 317 B1**

(12)                **EUROPEAN PATENT SPECIFICATION**

(45) Date of publication and mention
     of the grant of the patent:
     **18.03.2009   Bulletin 2009/12**

(51) Int Cl.:
     ***G01N 11/00*** (2006.01)

(21) Application number: **00941320.4**

(86) International application number:
     **PCT/US2000/015967**

(22) Date of filing: **09.06.2000**

(87) International publication number:
     **WO 2001/096832 (20.12.2001 Gazette 2001/51)**

(54) **QUASI-STATIC VISCOMETER**

QUASI-STATISCHES VISKOMETER

VISCOSIMETRE QUASI-STATIQUE

(84) Designated Contracting States:
     **AT BE CH CY DE DK ES FI FR GB GR IE IT LI LU
     MC NL PT SE**

(43) Date of publication of application:
     **02.04.2003   Bulletin 2003/14**

(73) Proprietor: **HONEYWELL INC.
     Morristown NJ 07960 (US)**

(72) Inventors:
     • **BONNE, Ulrich
       Hopkins, MN 55343 (US)**
     • **REZACHEK, Tom, M.
       Cottage Grove, MN 55016 (US)**
     • **KUBISIAK, David
       Chanhassen, MN 55317 (US)**

(74) Representative: **Hucker, Charlotte Jane
     Gill Jennings & Every LLP
     Broadgate House
     7 Eldon Street
     London EC2M 7LH (GB)**

(56) References cited:
     **US-A- 5 486 107**

     • **RUSSIAN PATENTS ABSTRACTS Section EI,
       Week 199326 Derwent Publications Ltd., London,
       GB; Class S03, AN 1993-212540 XP002901269 &
       SU 1 746 256 A (MORDASOV M M), 7 July 1992
       (1992-07-07)**

**Description**

BACKGROUND

The present invention pertains to viscosity detectors and particularly to delta-pressure-based sensors. More particularly, the invention pertains to viscosity sensors for determining the oxygen demand (for complete combustion) of a gaseous or liquid fuel for combustion purposes.

Existing and recently proposed quasi-static viscometers are either fluid (i.e., gas or liquid) density and pressure-dependent and costly (such as vibrating wire or quartz crystal-based viscometers). Other viscometers suffer from additional fluid property dependencies (e.g., those involving thermally-driven capillary flow), are prone to drift due to deteriorating and leaky valves (as in viscometers dependent on capillary flow driven by periodic refill from a source of pressurized gas, valve closure and decay observation), or depend on their orientation (as with the falling ball viscometer).

The proposed sensor measures a known property of fluids, viscosity. When applied to a combustion control system, it enables feed-forward operation and sensing in the mild pre-combustion environment; it is low-cost because the property can be simply proportional to the measured signal (in one preferred measurement approach) and it relates also simply to Wobbe number, oxygen demand or heating value of the fuel, so that the sensing error makes a relatively small contribution to the total combustion control error.

This invention involves the use or application of a known property, viscosity, to combustion control. It is also about using a preferred approach to viscosity measurement to that application.

Viscosity, $\eta$, may be known best for its linear relation to laminar volumetric flow (dV/dt) and pressure drop, $\Delta p$, in a capillary (of radius, $r_c$, and length, $L_c$), as shown in equation (1).

$$dV/dt \ = \ \pi \Delta p r_c^4 / (8 L_c \eta) \ (1)$$

One first notes the potential of viscosity as an individual property for combustion control when searching for low-cost means to compensate for variabilities in natural gas composition, and for a way to determine heating value without combustion, which includes analytical determination via correlations involving $k(T_1)$, $k(T_2)$ and $\eta$, i.e., in conjunction with other properties.

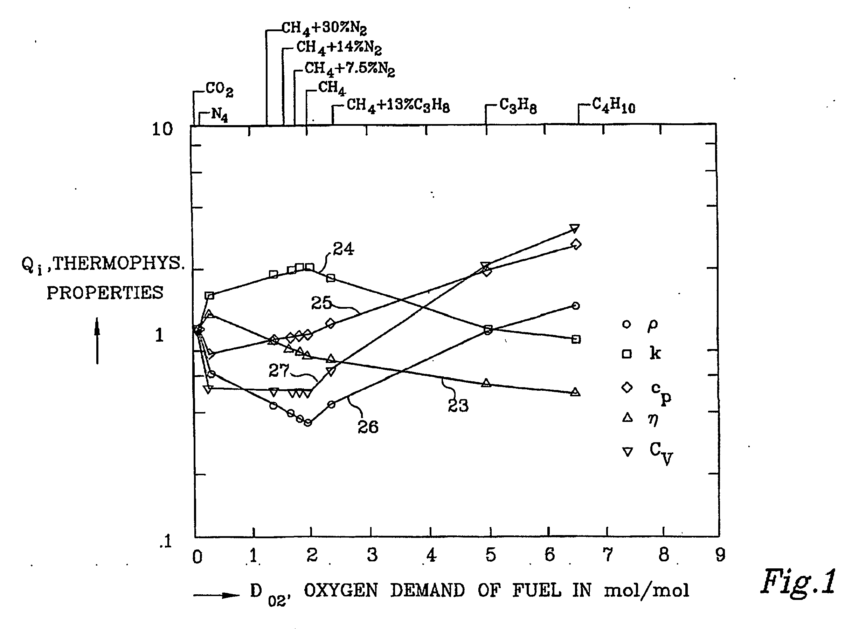

Here, the viscosity of the fuel, and, previously, the stack $O_2$ concentration were for indicating predicted or actual changes in the fuel's oxygen demand, $D_{O2}$. Figure 1 shows graphically a comparison between $\eta$, curve 23, and other fuel gas thermophysical $(Q_i)$ properties, i.e., $\rho$,density, curve 26; k, thermal conductivity, curve 24; $c_p$, specific heat, curve 25; and $C_v$, thermal anemometer correction factor, curve 27; and how well they correlate individually with oxygen demand of fuel, $D_{02}$. $\eta$ exhibits a most advantageous, monotonic decrease as $D_{02}$ increases, although $c_p$ appears promising as well. The $c_p$ value of noncombustible $CO_2$ (8.83 cal/(mol·K); 8.60 for $H_2O$) lies between that of $CH_4$ and $C_2H_6$ (8.50 and 12.42), but all $\eta$-values of noncombustible gases $O_2$, $N_2$, $CO_2$ (except $H_2O$) lie above that of $CH_4$.

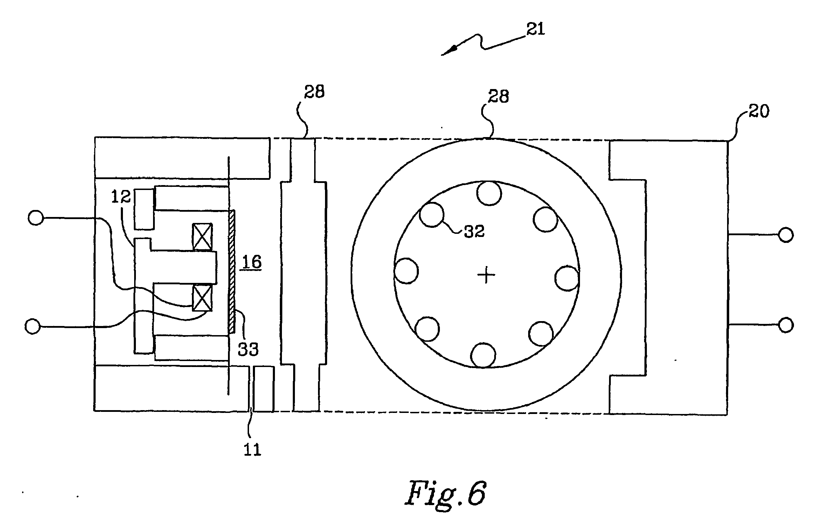

By including two or more fuel properties into a correlation with heating value or $D_{O2}$, the achievable accuracy increases significantly (note cited art below), but at the penalty of significant cost increases as well, because of the need for digital processing for determination of $c_p$. The above is based on the assumption that control of emissions and efficiency are prime goals of any combustion control; this is most closely achieved by operating under constant stack-$O_2$ or excess air, which in turn is met by maintaining a constant air flow and adjusting fuel flow in response to its composition variations, which change $D_{O2}$ and m*. Half of its density variations is taken care of by the factor m, as it affects all orifice- or venturi-controlled flow control situations. The aim of adjusting fuel flow to counter variabilities in Wb, Wobbe number, is similar but less correct (if one aims at conserving the A/F (air-to-fuel ratio) and emissions) and goes back to the definition of the Wobbe number, Wb = $\Delta$H/m*, with $\Delta$h = heating value rather than $O_2$ demand and m* = $(M_{gas}/M_{air})^{0.5}$. M is moles, Wb is closely aligned.with Bn (Bonne number = $D_{02}$/m*), as long as non-hydrocarbon fuel constituents such as $H_2$ and CO are absent. A correlation of $D_{O2}$ or Bn with viscosity may be determined with a formula $D_{O2} = A + B\eta^c$ or Bn = A' + B'$\eta^{0.5}$ , respectively. A and B are correlation coefficients and C is a correlation exponent. A' and B' are similarly correlation coefficients. The correlation is like that of natural law. Related information is in figure 6, page 21, of "Microsensors for Fluid Properties", by U. Bonne and D. Kubisiak, Scientific Honeyweller, Sensors Issue (1996). Additional information is in U.S. Patent 5,486,107 by U. Bonne, issued January 23, 1996 and entitled "Determination of Fuel Characteristics".

To illustrate the significance of the proposed, viscometer-based combustion control system, Table 1 compares some parameters relevant to the quality of a combustion control system based on thermal conductivity versus viscosity sensors. As shown, on all counts, the viscosity-based system lists more advantageous values such as smaller sensor output dependence on pressure and temperature but larger dependence fuel-gas composition or fuel concentration in a fuel + air mixture. The latter parameter was included to quantify the merits of direct measurement of thermo-physical properties of the fuel + air mixtures; as shown, measurement of viscosity or thermal conductivity in a premixed fuel + air mixture makes the pressure, temperature and humidity effects much larger than the sought fuel property effects. A similar case can be made for the measurement of $\eta$ or k in the stack gases.

Table 1 indicates advantages of viscosity versus thermal conductivity as $D_{O2}$ or $\lambda$, wavelength, sensors. $\lambda$= (actual fuel/air ratio) ÷ (stoichiometric air/fuel ratio). This table indicates that viscosity is approximately two times more sensitive to changes in $\lambda$ and $D_{O2}$ than thermal conductivity, but thirty percent less sensitive to variations in pressure and temperature. That means viscosity detection results in a several times more accurate sensor than thermal conductivity, for $D_{O2}$ or $\lambda$ measurement. The gas G20 is methane and G271 is a gas mixture of 74 percent methane and 26 percent nitrogen. p is pressure in bars, and T, temperature, is in degrees Celsius. W is the dependent variable, measuring the desired property ($\lambda$ or $D_{O2}$). Sensitivities of k and $\eta$ are relative to variability in nitrogen content of fuel mixed with air, $\lambda$, T, p and nitrogen content of pure fuel gas.

Table 1

| | | $\partial W/\partial x$ | |
| --- | --- | --- | --- |
| Dependence | Conditions | W=k | W=$\eta$ |
| - | - | % | % |
| 1. $\partial W(\lambda)/\partial$(fuel+air) | G20+air vs G271+air $\lambda$=1.05; 15°C | 0.2687 | -0.3532 |
| 2. $\partial W (\lambda) /\partial\lambda$ | $\lambda$=1.05 vs. 1.10 G20+air | 0.0930 | -0.1229 |
| 3. $\partial W (D_{O2}) /\partial$Gas | G20 vs G271 (26%$N_2$) 15°C; 1 bar | 6.7490 | -13.782 |
| 4. $\partial W(D_{O2})/\partial T$ | T=20 vs T=15°C; G20 | 1.6320 | 1.3860 |
| 5. $\partial W (D_{O2}) /\partial p$ | p=2 vs 1 bar; G20; | 0.1938 | 0.1043 |

For the most desirable property (k or $\eta$), the values of W for rows 1-3 should be the highest and 4-5 the lowest. Viscosity is obviously the preferred choice.

SU-A-1746256 relates to a turbulent sound-generator which is immersed in a liquid to be tested using a gas-transmitting tube and a turbulent flow of gas from the sound-sources formed through a layer of the liquid, forming a channel with fluid walls. During immersion of the sound-absorber to a critical depth, vibrations are formed, accompanied by a change of the form of the channel and the frequency of periodic change is determined by this feed of movement of the liquid wave along the channel. The amplitude of the sub-sonic vibrations determined by the amplitude of the liquid wave and acoustic amplitude-modulated vibrations pass through a microphone carrying information on the viscosity of the tested liquid. The amplitude and frequency of these vibrations are fixed by meters respectively and are used to assess the viscosity.

## SUMMARY OF THE INVENTION

The viscometer disclosed here does not rely on the availability of pressurized gas, a microsensor or on thermal drivers, and its output is independent of absolute pressure (to the extent that viscosity is). It is not sensitive to orientation and can be fabricated at low cost. The present viscosity sensor is used for determining the oxygen demand of various gaseous mixtures. It has a fluid volume-displacer, actuator or driver, such as a speaker membrane, and a pressure sensor or detector, such as a microphone, with a chamber or cavity and a controlled leak between the cavity and the ambient environment of the viscosity sensor. The driver, the leak and sensor electronics can be assembled from commercially available and inexpensive components. In sum, the sensor has low manufacturing costs, and good accuracy, reliability, intrinsic safety and long service life.

According to the present invention, there is provided a device for use in a quasi-static viscometer, as defined in claim 1.

## BRIEF DESCRIPTION OF THE DRAWING

Figure 1 is a graph of the relationship between oxygen demand of fuel and thermophysical properties of the fuel.

Figure 2 shows a viscometer.

Figures 3, 4 and 5 are graphs of an oscillatory volume and the delta pressure of a leaky cavity for three different gases.

Figures 6 and 7 show additional embodiments of the viscometer.

Figure 8 is a graph of drive voltage versus deflection for an actuator of a viscometer.

Figure 9 shows a viscometer and associated components for use in various applications.

## DESCRIPTION OF THE EMBODIMENT

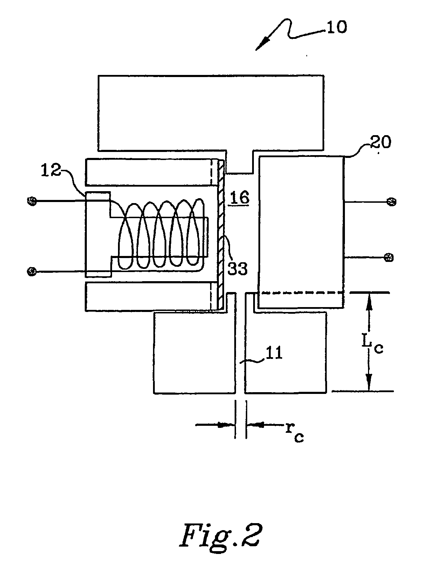

A viscometer 10 of figure 2 makes use of a linear relationship between laminar volumetric flow (dV/dt) through a controlled leak, for which we shall choose at first a capillary (C) 11, (of radius, $r_c$, and length, $L_c$, of capillary tube 11) and viscosity, $\eta$, for a pressure difference, $\Delta p$. The mathematical relationship of these parameters is shown in equation (2).

$$dV/dt = \pi \Delta p r_c^4 / (8 L_c \eta) \qquad (2)$$

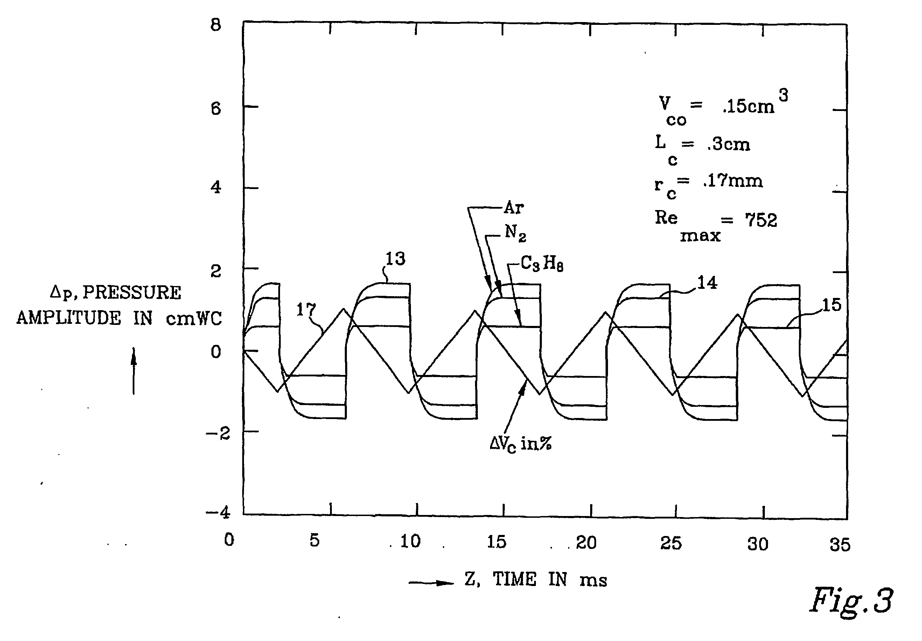

Instead of using a thermal source, a valve or a mechanical flow is used to induce a repeatable but time-dependent flow and enable the observation of a pressure drop (or rise), $\Delta p$, time constant, $\tau$, or phase lag, $\delta$. Viscometer 10 disclosed here is designed to induce a repeatable but time-independent flow to enable the observation of a steady $\Delta p$ when the rate of volumetric displacement by an actuator 12 and the actual leakage flow become equal for a few milliseconds (ms). This is sketched out and represented in Figures 2 and 3. The $\Delta p$ signals for three gases, Ar, N2 and $C_3H_8$, are marked as curves 13, 14 and 15, respectively, and the volume change $\Delta V_c$, in percent, of cavity 16, shown by saw-tooth curve 17, represents the volumetric change induced by actuator or driver 12.

If it can be assumed that the displacement of a sufficiently strong actuator is independent of the type of gas, actuator 12 can be driven in a "saw tooth" mode and at constant frequency, f, and the above equation (1) indeed establishes itself; then all types of gases will eventually reach their own $\Delta p$ in the chamber, but at the same capillary 11 flow rate, thus enabling the determination of each fluid's viscosity via measurement of $\Delta p$ and use of equation (2). If one designs viscometer 10 to meet the first two above-noted assumptions, the remaining question is whether there will be enough time in one half-cycle to establish the above quality. If the capillary radius $r_c$ is too small, the $\Delta p(t)$ will also be a saw-tooth-like function (one may neglect adiabatic heating effects for small $\Delta V_c$); but if capillary 11 is large enough, its flow and $\Delta p$ will only increase until dV/dt = $\Delta V_c f/2$, regardless of the viscosity of the gas in the viscometer 10, and then remain at that value until the end of the saw-tooth 17 period. The viscosity then results from equation (3).

$$\eta = \pi \Delta p r_c^4 / (4 \Delta V_c f L_c) \qquad (3)$$

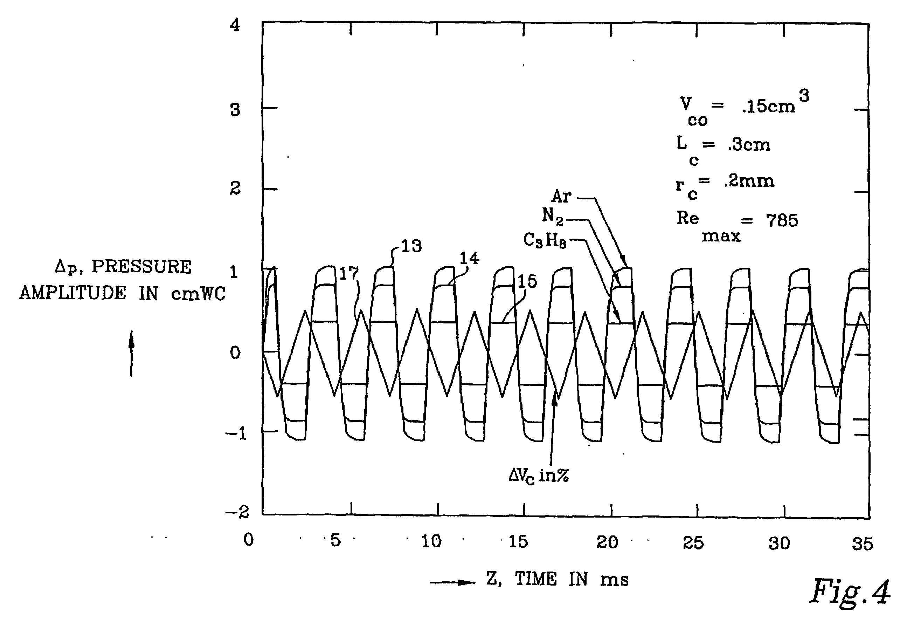

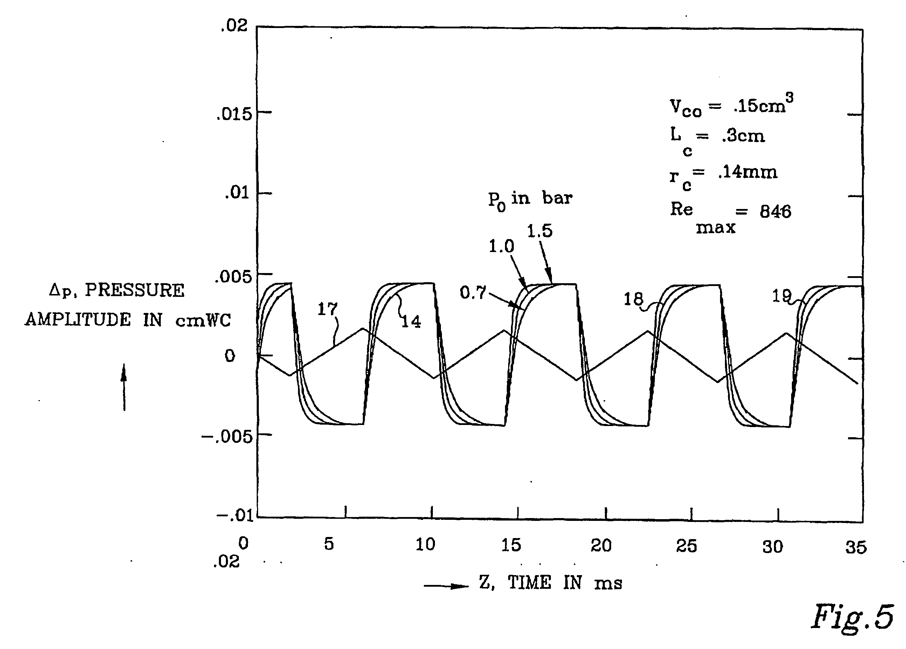

This relationship is illustrated by the results of calculations with a numerical model in Figures 3 and 4, whereby for a given $r_c$, $L_c$ and f, the cavity volume would be incremented by a small amount corresponding to a time step, $\Delta z = 0.1$ ms, which would change the cavity pressure by an amount corresponding to PV = nRT, which in turn would start the flow through the capillary and remove some of the pressure change during $\Delta z$, until a balance between dV/dt = $dV_c/dt$ = $V_c f/2$ and $\Delta p$ is reached. These figures show that: (1) steady $\Delta p$ values can be achieved towards the end of the saw-tooth periods; (2) the values of such steady $\Delta p$ periods are proportional to the viscosities of the indicated gases $C_3H_8$, $N_2$ and Ar (83, 178 and 224 $\mu$P at 20°C and 1 atm, respectively); (3) the $R_v = \Delta V_c/V_c$, $r_c$ and $L_c$ values need to be and can be chosen to both achieve a steady $\Delta p$ period and laminar flow (Re < 2300) in the capillary, as indicated by the Reynolds Number, Re = $2rv\rho/\eta$, for the frequencies of 124 and 324 Hz, and the lowest $\eta/\rho$-gas, which was propane in Figures 3 and 4; the 324 Hz frequency was chosen to be away about equally from higher harmonics of 50 and 60 Hz; and (4) the time constants to reach the steady $\Delta p$-period are longer for higher viscosity fluids and lower pressure gases. The time constant results are illustrated in Figure 5.

Viscosity, $\eta$, is an indication of oxygen demand, $D_{O2}$, of the fluid.

Figure 3 is a graph of $\Delta p$, pressure amplitude in cm water column (WC) versus z or time in ms. This graph shows an oscillatory volume 17 and $\Delta p$ of a leaky cavity 16 for three gases Ar, $N_2$ and $C_3H_8$, as represented by curves 13, 14 and 15, respectively. The oscillatory volume 17 of cavity 16 is the result of electrical input to actuator 12. The pressure sensor changes, as indicated by curves 13, 14 and 15, are detected by sensor 20. These plots were taken at 22°C and a pressure of 0.7 bar. The frequency, f, is 132 Hz. $R_v$ is one percent. The pressure equilibration times to 63% of the final $\Delta p$, $\tau$, are 0.40, 0.23 and 0.153 ms for the three gases, respectively. The cavity volume at rest, $V_{co}$, is 0.15 cm³, and capillary length $L_c$ and radius $r_c$ are at 0.3 cm and 0.17 cm, respectively. The maximum Reynolds number,

$Re_{max}$, is 752.

Figure 4 is a graph of $\Delta p$, pressure amplitude in cm WC versus time in ms, z. This graph shows an oscillatory volume 17 and $\Delta p$ of leaky cavity 16 for three gases Ar, $N_2$ and $C_3H_8$, as represented by curves 13, 14 and 15. These plots were taken at 22° C and a pressure of 0.7 bar. Frequency f is 323 Hz, which is about 2.447 times faster than f for figure 2. $R_v$ is 0.5%, $\Delta z$ is 0.05 ms and the linear excitation, $\tau$, is 0.18 ms. $V_{co}$ and $L_c$ are the same as for figure 3. The capillary radius, $r_c$, is larger at 0.2 mm. The maximum Reynolds number is 785. Note that the amplitudes of corresponding curves 13, 14 and 15 are about half of those for the curves in figure 3. However, the amplitude differences between curves 13, 14 and 15 appear to be more distinguishable in figure 4 than figure 3.

Figure 5 is a graph of $\Delta p$, pressure amplitude in cm WC versus Z, time in ms. This graph shows an oscillatory volume 17 and $\Delta p$ of leaky cavity 16 for $N_2$ at three different absolute pressures, $p_a$. Curve 14 is for $N_2$ at 0.7 bar, curve 18 is for $N_2$ at 1.0 bar and curve 19 is for $N_2$ at 1.5 bar. This data was taken from device 10 at 22° C and a frequency of 122 Hz, and $R_v$ is equal to 1%. $V_{co}$ is 0.15 cm$^3$, $L_c$ is 0.3 cm, $r_c$ is 0.14 mm and $Re_{max}$ is at 846. These curves in figure 5 show that the $\Delta p$-equilibria reached are independent of absolute pressure. But they also show that the time constants to reach equilibrium, $\tau$, are pressure dependent: time constants of about 0.29, 0.23 and 0.194 ms were derived for the pressures of 0.7, 1.0 and 1.5 bar, respectively, which may also serve to determine absolute pressure after viscosity has been determined: $p_a \tau^2 \approx$ constant.

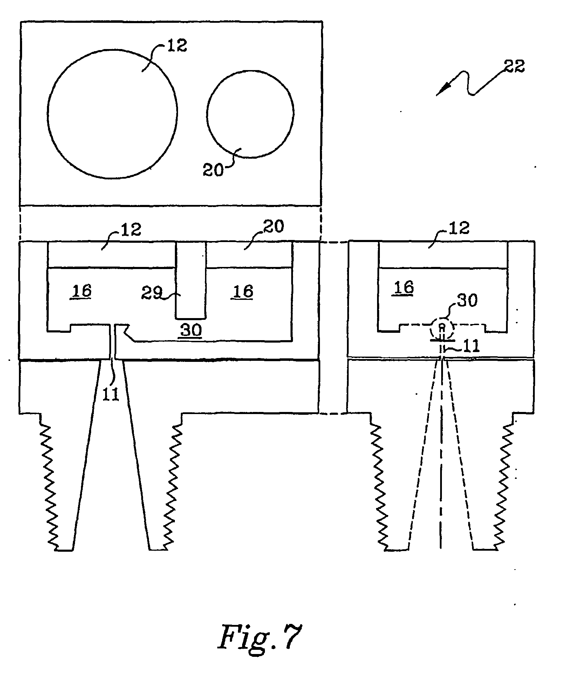

To implement viscometer 10, one needs to consider volumetric drivers 12, pressure sensors 20 and possible system limitations. Figures 6 and 7 show additional embodiments 21 and 22, respectively, featuring interfaces or coupling devices 28 and 29 that may be baffles or at least partial barriers between drivers 12 and sensors 20. Barrier 28 or 29 is for preventing transport from driver 12 to sensor or detector 20 any physical energy (i.e., mechanical, electrical, and/or thermal) which may hinder, delay or inhibit the transfer of $\Delta p$ information from driver 12 to detector 20. Baffle 28 or 29 may prevent such possible distortion or dilapidation by effecting diffusion, attenuation or other appropriately affecting function.

Viscometer 21 has a baffle 28 situated in cavity 16 between actuator 12 and sensor 20. Baffle or diffuser plate 28 has apertures or holes 32 so that driver 12 can affect sensor 20 via the tested gas and apertures 32. The gas enters cavity 16 via capillary 11. A.front view of diffuser plate 28 is shown to illustrate apertures 32. There is only one plate 28, which is situated facing diaphragm 33. Viscometer 22 has a baffle, bar or damping channel 29 situated in cavity 16 between actuator 12 and sensor 20. A passage or hole 30 provides for passage of the tested gas so that driver 12 can affect sensor 20, for viscosity determination of the gas. The controlled cavity leak is though capillary opening 11.

Actuators may be of several kinds. To generate constant rate of volumetric expansion or contraction, one can consider a bulk (piezoelectric) PZT expansion, PZT bimorph actuators as used in tweeters and electromagnetic speakers as drivers 12. Volumetric expansion of piezoelectric transducers is attractive because of the large forces involved, which would not be affected by changes in gas density, although the displacements would be very small. Both PZT expansion and PZT bimorph actuators would need to be temperature-compensated, which would not be required in the instance of electromagnetic speakers.

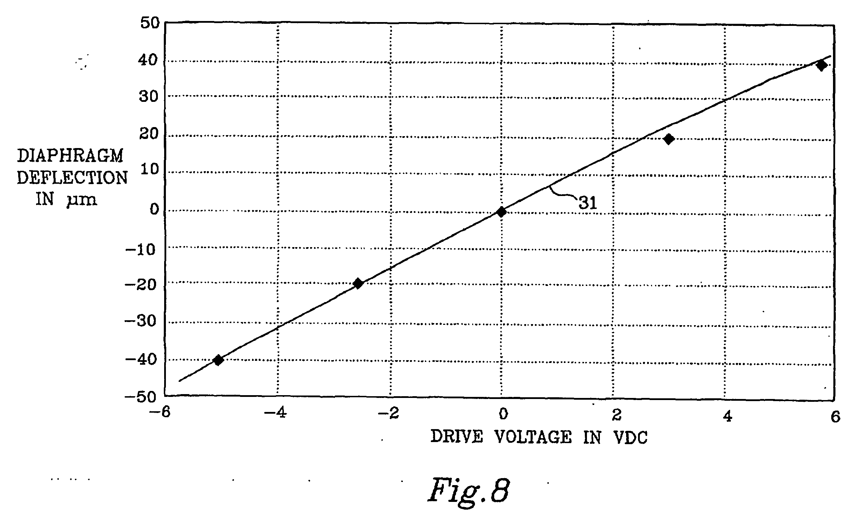

Measured center deflections were made of a bimorph actuator (PZT/brass lamination by Mallory Sonalert of Indianapolis, IN, at $0.55/each at a 25-99 quantity) consisting of a 15 mm OD/0.11 mm-thick brass support + a 10 mm OD/0.11 mm-thick PZT + 9 mm OD/0.02 mm-thick Ag electrode film. Under the rated voltage of $\leq$ 25 V, the center deflection was about 0.8 $\mu$m/VRMS. Center deflection measurements were also made of an electromagnetic speaker (BRT1209P-01, from Int'1. Components Co. of Melville, NY, at a price for a quantity of 25-99 at $0.60/each) and rated at for a maximum input of 1-2 VDC and 10 mA maximum. The center displacement of this 50 $\mu$m-thick speaker disc was found to be fairly linear with input voltage (see curve 31 of Figure 8). The displacement amounted to about 7.5 $\mu$m/ volt from -6 to +6 VDC. This displacement per volt (V) is thus about 10 times greater than the one from a PZT bimorph. Support of the speaker membrane 33 merits some careful consideration (note membrane 33 of designs 10 and 21 of Figures 2 and 6, respectively).

If chamber or cavity 16 is sealed to eliminate leaks across speaker membrane 33, then the side opposite to its $V_c$ side needs to be opened to an ambient/external fluid to avoid anomalous effects when the ambient pressure or temperature changes. If the volume on the membrane 33 side opposite of the cavity 16 side is not sealed leaktight from cavity 16 (as in BRT1206P-01), then it competes with capillary 11 and/or the leak functions as an orifice.

Sensors may be of several kinds. Another function needed for viscometer 10 is the $\Delta p$ sensor 20 between ambient and $V_c$. Microphones may be the lowest-cost choice to meet that need. The ideal sensor 20 would be a rigid microphone, i.e., lack of influence on $V_c$ via deformation, with a frequency-independent output. An electret microphone (by Panasonic of Secaucus, NJ, or Gentex of Carbondale, PA, with a deformable membrane) was used in phase lag measurements. It provided an unamplified output of 200 mV for about 50-60 Hz pressure variations of $\leq$ 1.2 cm of $H_2O$. The noise level was at 2 mV, which is equivalent to $(1.2/1000)(2/200) \cdot 10^6 = 12$ ppm of pressure change in cavity 16, or 0.012 cm of $H_2O$.

An electromagnetic speaker (BRT1209P-01 by Int'1. Components Co.), was tested as a sensor 20, back-to-back to a similar unit serving as a driver 12. However, their magnetic fields interfered, and thus shielding would be needed for good results.

A MICRO SWITCH 24PC pressure sensor chip, from Honeywell Inc. of Freeport, IL, mounted and sealed on a TO5 header (over a center hole to avoid any back-pressure build-up) served well as a sensor 20, in operation with one of the BRT1206P-01 speakers serving as an actuator 12. It was verified that the sensor 20 output followed the shape of the driver 12 excitation (sine, square or saw-tooth shape) with capillary 11 plugged. After unplugging the capillary, a balance between rate of cavity 16 volume change and capillary 11 flow was achieved, demonstrating the invention. During this dynamic balance, the established $\Delta p$ was representative of the fluid viscosity. The L/D ratio of the capillary 11 tube should be greater than four. The capillary 11 inlet should be smooth internally and at the edges to minimize turbulence.

Experiments with the same driver 12, but a piezoelectric speaker serving as sensor 20, yielded a pressure-independent relationship that was as expected theoretically, $\eta \approx \Delta G p^0$ (where $\Delta G$ = pressure sensor output and p = absolute pressure) within a $\pm 5\%$ scope measurement error. This viscosity sensor consisted of simply epoxying an available \$0.60 driver 12 and \$0.55 sensor 20 back-to-back, and being operated at 60 Hz. In summary, very low-cost, off-the-shelf components show that fabrication of the present viscometer 10, 21 or 22 can be very cost-effective.

Viscometer 10 may have several limitations. First, one possible limitation is head pressure due to flow reversal. As the frequency of a flow driver increases, the inertial pressure generated at each reciprocating flow reversal increases. In order to stay away from such effects, one may calculate the frequency at which the capillary 11 pressure drop, $\Delta p_c$, would equal the inertial pressure drop, $\Delta p_i$, as shown by equation (4).

$$\Delta p_C = (\Delta V_c f/2) \cdot 8\eta L_c/(\pi r^4) \text{ and } \Delta p_i = 2\rho v^2/2 =$$

$$\rho (\Delta V_C f/(2\rho r^2))^2 (4)$$

With the assumption that the steady volume flow in each direction, $\Delta V$-f/2, generates an average velocity, $v = \Delta V_c f/(2\pi r^2)$, and an inertial pressure pulse upon reversal of $2\rho v^2/2$, then for $\Delta p_c = \Delta p_i$, one gets

$$8\eta L_c = \rho \Delta V_c f/(2\pi) \text{ and } f = 16\pi\eta L_c/(\rho\Delta V_c). \qquad (5)$$

For $\eta$ = 0.000178 g/(cm·s) or $v = \eta/\rho$ = 0.153 cm²/s for $N_2$ at 20°C and 1 bar, $L_c$ = 1 cm and $V_c$ = 0.0001 cm³, one gets f = 77 kHz. For most applications, one will therefore be able to neglect this effect because it is generally small at low frequencies and it only occurs at each flow reversal.

Second, the appearance of turbulence in a capillary 11 tube needs to be avoided. For the above example, with r = 0.008 cm, one gets Re = $2r_c v/v$ = 1909 for $N_2$ (but 6935 for propane). However, appropriately combined changes in $r_c$, $\Delta V_c$ and f, provide a laminar range that is wide indeed. To minimize the onset of turbulence, the edges at the ends of capillary 11 should be made smooth.

Third, The stability of the actuator, $\Delta p$, sensor and controlled leak (no plugging with time) are clearly critical to long-term, reliable service. The way to maintain stable displacement via circuitry, stable leak via a multiplicity of leaks in porous plate, and self-checking the sensor for proper operation and accuracy are recommended to overcome stability limitations.

The components of the quasi-static viscometer 10 and their costs, include a saw-tooth generator at \$0.4, a speaker (10-15 mm diameter) at \$0.22-0.25 (from DAI Ltd.), a microphone (6-10 mm diameter) at \$0.22-0.25, a microphone amplifier and an analog-to-digital converter (A/D) for a digital output, at about \$2.00, one or more 0.2-0.4 mm holes/capillaries of 3-6 mm in length, or equivalent controlled leaks made of porous materials, at \$0.10, plus assembly, calibration and miscellaneous materials at about \$3.00. Thus, the total cost of the sensor may be less than \$6.00, so that its use and business potential is great.

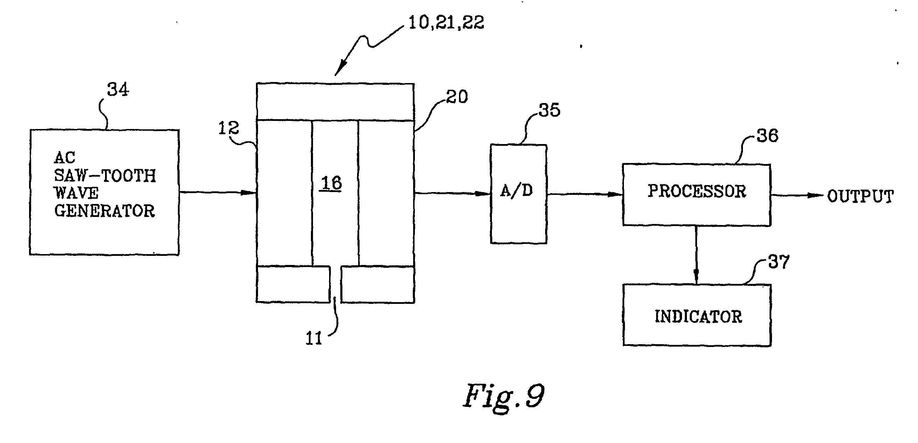

Features of the invention involve the combination of capillary 11 flow (known to be proportional to viscosity) to or from a cavity 16, an electro-mechanical fluid displacer/actuator 12 and a saw-tooth electronic drive 34 to enable the $\Delta p$ in cavity 16 to stabilize during at least one of the two periods of each AC actuation cycle. The measurement of the established $\Delta p$ at the end of (at least one or) each saw-tooth period is an indication of the desired viscosity. It is the $\Delta p$ (but not $\Delta p/p_a$) which is largely independent of absolute pressure, $p_a$. $\Delta p$ is sensed by sensor 20, which provides an output to an analog-to-digital converter 35, as shown in figure 9. The digital output goes to processor 36 for processing. Processor 36 has a digital or analog output that may be provided to some apparatus such as a combustion control or regulator. Also, an output is provided to indicator 37 that may provide readable information about the directly measured viscosity, as well as correlated properties such as oxygen demand or other parameters of the tested gas. The output of sensor 20, instead, may go directly to an analog indicator, processor or interface.

An embodiment may have an actuator 12 that is a low-voltage, electro-magnetic earphone speaker, rather than a piezo-electric tweeter or electro-static speaker, and in which the $\Delta$p sensor 20 is a microphone based on either piezo-electric (preferred), piezo-resistive, electro-magnetic, electret, carbon-contact or capacitance effects. The present invention may sense not only viscosity but also absolute pressure by further processing the determined viscosity and the initial time constant or phase lag between actuator 12 input and sensor 20 output, as shown in figures 3-5.

Viscometers 10, 21 and 22 have advantages over related art viscometers based on vibrating wires and quartz crystal oscillators, having pressure decays or phase lags, because they have no dependence on absolute pressure. Viscometers 10, 21 or 22 also has advantages over viscometers based on thermal excitation, because it has no dependence on thermal conductivity or specific heat of the fluid being measured. It also has advantages over traditional viscometers based on capillary flow driven by a constant source of pressurized fluid, or based on timing of the fall of an object, in that there is no need for a costly source of constant pressure fluid, constant gravity magnitude and direction, or equipment for providing and measuring the falling object. The present viscometer is not sensitive to its mounting orientation. As noted above, besides viscosity, the present device can also be used to sense absolute pressure by using the initial time constant or phase lag between actuator input and sensor output, which is a feature not available from related art viscometers.

Other embodiments and variants of the present invention, not disclosed here, are covered by the claims and only limited in scope by the claims, which includes all equivalents thereof.

## Claims

1. A device for use in a quasi-static viscometer (10) comprising:

   a driver (12) having a diaphragm (33),
   a sensor (20) having a diaphragm situated at a first distance from the diaphragm (33) of said driver (12),
   **characterised in that** a fitting is formed between and around peripheries of the diaphragms of said driver (12) and sensor (20) to form a cavity (16) between the diaphragms, and
   an opening (11) is formed on said fitting to provide a leak for the cavity (12).

2. The device (10) of claim 1, further comprising an interface situated in the cavity (16) between the diaphragm (33) of said driver (12) and the diaphragm of said sensor (20), wherein the cavity (16) is divided into two subcavities.

3. The device (10) of claim 2, wherein said interface has a plurality of holes.

4. The device (10) of claim 3, wherein:

   said driver (12) is a speaker, and
   said sensor (20) is a microphone.

5. The device (10) of claim 4, further comprising:

   an signal generator connected to said driver (12), and
   a processor (36) connected to said sensor (20).

6. The device of claim 5, wherein said processor (36) processes signals from said sensor (20) into indications of viscosity of a fluid within the cavity (16).

7. The device of claim 6, wherein said processor (36) processes the indications of viscosity into indications of heating value, $D_{02}$, of the fluid within the cavity (16).

8. The device of claim 7, wherein:

   said opening is a capillary tube (11) having a length, $L_c$ and a radius, $r_c$,
   said signal generator outputs a signal having a frequency, f, to said driver,
   said driver (12) causes a volume, $\Delta V_c$, of a fluid to enter and leave the cavity (16) at a pressure variation, $\Delta$p, and
   the viscosity, $\eta$, of the fluid within the cavity (16) is equal to $(\pi \Delta p r_c^4) / (4 \Delta V_c f L_c)$.

**Patentansprüche**

1. Vorrichtung zur Verwendung in einem Viskometer (10), umfassend:

   einen Treiber (12) mit einer Membran (33),
   einen Sensor (20) mit einer Membran, die sich in einem ersten Abstand von der Membran (33) des Treibers (12) befindet,
   **dadurch gekennzeichnet, daß** zwischen und um Peripherien der Membranen des Treibers (12) und des Sensors (20) eine Passung gebildet wird, um einen Hohlraum (16) zwischen den Membranen zu bilden, und eine Öffnung (11) an der Passung zur Bereitstellung eines Lecks für den Hohlraum (16) gebildet wird.

2. Vorrichtung (10) nach Anspruch 1, ferner mit einer in dem Hohlraum (16) zwischen der Membran (33) des Treibers (12) und der Membran des Sensors (20) befindliche Grenzfläche, wobei der Hohlraum (16) in zwei Teilhohlräume unterteilt wird.

3. Vorrichtung (10) nach Anspruch 2, wobei die Grenzfläche mehrere Löcher aufweist.

4. Vorrichtung (10) nach Anspruch 3, wobei
   der Treiber (12) ein Lautsprecher ist und
   der Sensor (20) ein Mikrofon ist.

5. Vorrichtung (10) nach Anspruch 4, ferner umfassend:

   einen mit dem Treiber (12) verbundenen Signalgenerator und
   einen mit dem Sensor (20) verbundenen Prozessor (36).

6. Vorrichtung nach Anspruch 5, wobei der Prozessor (36) Signale aus dem Sensor (20) zu Indikationen für die Viskosität eines Fluids in dem Hohlraum (16) verarbeitet.

7. Vorrichtung nach Anspruch 6, wobei der Prozessor (36) die Indikationen für die Viskosität zu Indikationen für den Brennwert $D_{02}$ des Fluids in dem Hohlraum (16) verarbeitet.

8. Vorrichtung nach Anspruch 7, wobei
   die Öffnung eine Kapillarröhre (11) mit einer Länge $L_c$ und einem Radius $r_c$ ist,
   der Signalgenerator ein Signal mit einer Frequenz f an den Treiber ausgibt,
   der Treiber (12) bewirkt, daß ein Volumen $\Delta V_c$ eines Fluids unter einem Druckwechsel $\Delta p$ in den Hohlraum (16) eintritt und diesen verläßt, und
   die Viskosität η des Fluids in dem Hohlraum (16) gleich $(\pi\Delta pr_c^4)/(4AV_cfL_c)$ ist.

**Revendications**

1. Dispositif destiné à être utilisé dans un viscosimètre (10) quasi statique comprenant :

   un amplificateur (12) ayant un diaphragme (33),
   un capteur (20) ayant un diaphragme situé à une première distance du diaphragme (33) dudit amplificateur (12),
   **caractérisé en ce qu'**un ajustage est formé entre les périphéries du diaphragme dudit amplificateur (12) et dudit capteur (20) et autour de celles-ci de façon à former une cavité (16) entre les diaphragmes, et
   une ouverture (11) est formée sur ledit ajustage pour fournir une fuite pour la cavité (12).

2. Dispositif (10) selon la revendication 1, comprenant en outre une interface située dans la cavité (16) entre le diaphragme (33) dudit amplificateur (12) et le diaphragme dudit capteur (20), dans lequel la cavité (16) est divisée en deux cavités secondaires.

3. Dispositif (10) selon la revendication 2, dans lequel ladite interface a une pluralité de trous.

4. Dispositif (10) selon la revendication 3, dans lequel :

ledit amplificateur (12) est un haut-parleur, et
ledit capteur (20) est un microphone.

5. Dispositif (10) selon la revendication 4, comprenant en outre :

un générateur de signaux connecté audit amplificateur (12), et
un processeur (36) connecté audit capteur (20).

6. Dispositif (10) selon la revendication 5, dans lequel ledit processeur (36) traite les signaux provenant dudit capteur (20) en des indications de viscosité d'un fluide à l'intérieur de la cavité (16).

7. Dispositif (10) selon la revendication 6, dans lequel ledit processeur (36) traite les indications de viscosité en des indications d'une valeur de chauffage $D_{02}$ du fluide à l'intérieur de la cavité (16).

8. Dispositif (10) selon la revendication 7, dans lequel :

ladite ouverture est un tube capillaire (11) ayant une longueur $L_c$ et un rayon $r_c$,
ledit générateur de signaux fournit un signal ayant une fréquence f audit amplificateur,
ledit amplificateur (12) fait qu'un volume $\Delta v_c$ d'un fluide entre dans la cavité (16) et la quitte à une variation de pression $\Delta p$, et
la viscosité $\eta$ du fluide à l'intérieur de la cavité (16) est égale à $(\pi \Delta p r_c^4) / (4 \Delta V_c f L_c)$.

Fig.1

*Fig.2*

Fig.3

EP 1 297 317 B1

Fig.4

Fig.5

*Fig.6*

EP 1 297 317 B1

*Fig. 7*

*Fig.8*

EP 1 297 317 B1

EP 1 297 317 B1

*Fig.9*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5486107 A, U. Bonne

- SU 1746256 A